# EUROPEAN PATENT APPLICATION

(11) **EP 2 853 884 A1**
(43) Date of publication of application: **01.04.2015**
(21) Application number: 13185938.1
(22) Date of filing: 25.09.2013
(51) Int. Cl.: G01N 21/64, G01N 33/28, B82Y 15/00

(54) **A technique for determining metals in oil samples obtained from lubricating oil of machines**

(71) Applicant: Siemens Aktiengesellschaft, 80333 München (DE)
(72) Inventor: Minasamudram, Rupa, 560008 Bangalore (IN); Perumal, Venkateswaran, 560100 Bangalore (IN)

(57) **Abstract**

An oil testing method (1000) and an oil testing device (1) for determination of at least one metal (6, 8) in an oil sample (3) obtained from a lubricant oil of a machine are presented. In the oil testing method (1000), a quantum dot (5, 7) is added to the oil sample (3). The quantum dot (5, 7) is adapted to specifically attach with the metal (6, 8). The quantum dot (5, 7) and the metal (6, 8) are capable of generating a fluorescence resonance energy transfer (FRET) when the quantum dot (5, 7) is attached with the metal (6, 8). The quantum dot (5, 7) in the oil sample (3) is excited by an electromagnetic radiation (12) incident on the oil sample (3). Finally, from the oil sample (3) a fluorescence spectrum (95, 97) corresponding to the quantum dot (5, 7) so excited is detected. The metal (6, 8) is determined by observing a quenching in the fluorescence spectrum (95, 97) corresponding to the quantum dot (5, 7).

## Description

The present invention relates to a technique for lubricant oil testing, and more particularly to a method and a device for determining at least one metal in an oil sample.

Lubricant oil is a key component used in industries in order to ensure proper functioning of machines such as industrial machinery for example turbines, gears, bearings, compressors, and so forth. The parts of the machines undergo wear for several reasons such as misalignment of machine parts, manufacturing or functional defects in the machine parts, improper operation and maintenance of the machine parts, insufficient lubrication of the machine parts, and so on. The wear makes the machine parts weak and shortens their usable lifetime. The wear of the machine parts is manifested in the lubricating oil by presence of metals that wear out from the machine parts. The metals that wear out from the machine parts include Iron (Fe), Copper (Cu), Lead (Pb), Tin (Sn), etc. Thus by analysing the lubricating oil for determining metals in the lubricant oil helps diagnose important information regarding condition and functioning of the machine and its parts.

Currently, oil samples are manually drawn and sent to specialized laboratories, where they are analyzed by complex analytical instrumentation on a periodic basis. Periodic need of laboratory analysis makes the process time consuming, labor intensive and expensive. In order to reduce manual labor, time and operational cost associated with the laboratory analyses, a simple technique for determining metals in the oil samples is required.

It is therefore an object of the present invention to provide a simple technique for determining metals in oil samples obtained from lubricating oil of machines.

The object is achieved by an oil testing method according to claim 1 and an oil testing device according to claim 10 of the present technique.

According to a first aspect of the present technique, an oil testing method for determination of at least one metal in an oil sample is presented. The oil sample is obtained from a lubricant oil of a machine. In the oil testing method, a quantum dot is added to the oil sample. The quantum dot is adapted to specifically attach with the metal. The quantum dot and the metal are capable of generating a fluorescence resonance energy transfer when the quantum dot is attached with the metal. Subsequently, the quantum dot in the oil sample is excited by an electromagnetic radiation incident on the oil sample. Finally, from the oil sample a fluorescence spectrum corresponding to the quantum dot so excited is detected. The metal is determined by observing a quenching in the fluorescence spectrum corresponding to the quantum dot. Thus a presence of the metal in the oil sample is determined.

In an embodiment, the oil testing method further includes determining a concentration of the metal in the oil sample. In determining the concentration of the metal in the oil sample, an intensity of an electromagnetic signal in the fluorescence spectrum corresponding to the quantum dot is identified. The electromagnetic signal corresponds to the quantum dot attached with the metal. Subsequently, the intensity so identified is compared with a set of reference intensities. Each of the reference intensities in the set corresponds to a unique concentration value of the metal in the oil sample. Thus the concentration of the metal in the oil sample is determined.

In another embodiment, the oil testing method further includes determining an additional metal in the oil sample. In determining the additional metal in the oil sample, an additional quantum dot is added to the oil sample. The additional quantum dot is adapted to specifically attach with the additional metal. The additional quantum dot and the additional metal are capable of generating a fluorescence resonance energy transfer when the additional quantum dot is attached with the additional metal. Subsequently, the additional quantum dot in the oil sample is excited by the electromagnetic radiation incident on the oil sample. Finally, from the oil sample a fluorescence spectrum corresponding to the additional quantum dot so excited is detected. The additional metal is determined by observing a quenching in the fluorescence spectrum corresponding to the additional quantum dot. Thus a presence of the additional metal in the oil sample is determined. This makes the method more universal since it is not limited to the determination of only one particular metal.

In another embodiment of the oil testing method, the quantum dot and the additional quantum dot are excited simultaneously. Thus the determination of the metal and the additional metal is initiated at the same time allowing multiplexing through usage of the quantum dot and the additional quantum dot, therewith reducing the time to be invested into determining the metal and the additional metal.

In another embodiment of the oil testing method, the fluorescence spectrum corresponding to the quantum dot and the fluorescence spectrum corresponding to the additional quantum dot are detected simultaneously. This allows multiplexing through usage of the quantum dot and the additional quantum dot, therewith reducing the time to be invested into determining the metal and the additional metal.

In another embodiment, the oil testing method further includes determining a concentration of the additional metal in the oil sample. In determining the concentration of the additional metal in the oil sample, an additional intensity of an additional electromagnetic signal in the fluorescence spectrum corresponding to the additional quantum dot is identified. The additional electromagnetic signal corresponds to the additional quantum dot attached with the additional metal. Subsequently, the additional intensity so identified is compared with a set of additional reference intensities. Each of the additional reference intensities in the set corresponds to a unique concentration of the additional metal in the oil sample. Thus the concentration of the additional metal in the oil sample is determined.

In another embodiment of the oil testing method, the additional quantum dot includes a core of semiconductor material and a shell surrounding the core. The shell includes a binding material for specifically attaching with the additional metal. Thus any readily available quantum dot can be modified into having specificity for a given metal, e.g. the additional metal, and it can be easily used in the method.

In another embodiment of the oil testing method, the quantum dot includes a core of semiconductor material and a shell surrounding the core. The shell includes a binding material for specifically attaching with the metal. Thus any readily available quantum dot can be modified into having specificity for a given metal and readily used in the method.

In another embodiment of the oil testing method, the electromagnetic radiation incident on the oil sample is an Ultraviolet (UV) radiation. The UV radiation provides an easy and readily available way of exciting the quantum dot and the additional quantum dot, if available.

According to a second aspect of the present technique, an oil testing device for determination of at least one metal in an oil sample by using a quantum dot is presented. The quantum dot is adapted to specifically attach with the metal. The quantum dot and the metal are capable of generating a fluorescence resonance energy transfer when the quantum dot is attached with the metal. The oil testing device includes a sample port for receiving the oil sample and the quantum dot, an electromagnetic radiation source, a detector and a processor. The electromagnetic radiation source is adapted to generate an electromagnetic radiation and to excite the quantum dot in the oil sample by directing the electromagnetic radiation onto the oil sample. The detector is adapted to detect from the oil sample a fluorescence spectrum corresponding to the quantum dot so excited. The processor is adapted to determine a quenching in the fluorescence spectrum corresponding to the quantum dot wherein the metal is determined by the quenching so determined in the fluorescence spectrum corresponding to the quantum dot. Thus the oil testing device provides a simple device for determining a presence of the metal in the oil.

In an embodiment of the oil testing device, the processor is adapted to identify an intensity of an electromagnetic signal in the fluorescence spectrum corresponding to the quantum dot so detected. The electromagnetic signal corresponds to the quantum dot attached to the metal. The processor is also adapted to compare the intensity so identified with a set of reference intensities. Each of the reference intensities in the set corresponds to a unique concentration value of the metal in the oil sample. Thus the oil testing device is enabled to determine a concentration of the metal in the oil.

In another embodiment, the oil testing device is adapted to determine an additional metal in the oil sample by using an additional quantum dot. The additional quantum dot is adapted to specifically attach with the additional metal. The additional quantum dot and the additional metal are capable of generating a fluorescence resonance energy transfer when the additional quantum dot is attached with the additional metal. The sample port is adapted to receive the additional quantum dot. The electromagnetic radiation source is adapted to excite the additional quantum dot in the oil sample by directing the electromagnetic radiation onto the oil sample. The detector is adapted to detect from the oil sample a fluorescence spectrum corresponding to the additional quantum dot so excited. The processor is adapted to determine a quenching in the fluorescence spectrum corresponding to the additional quantum dot and wherein the additional metal is determined by the quenching so determined in the fluorescence spectrum corresponding to the additional quantum dot. Thus the oil testing device is enabled to determine a presence of the additional metal in the oil. This makes the method more universal since it is not limited to the determination of only one particular metal.

In another embodiment of the oil testing device, the detector is adapted to detect the fluorescence spectrum corresponding to the quantum dot and to detect the fluorescence spectrum corresponding to the additional quantum dot simultaneously. This obviates the need for multiple detectors and the time to be invested into determining the metal and the additional metal is reduced.

In another embodiment of the oil testing device, the processor is adapted to identify an additional intensity of an additional electromagnetic signal in the fluorescence spectrum corresponding to the additional quantum dot. The additional electromagnetic signal corresponds to the additional quantum dot attached to the additional metal. The processor is also adapted to compare the additional intensity so identified with a set of additional reference intensities. Each of the additional reference intensities in the set corresponds to a unique concentration of the additional metal in the oil sample. Thus the oil testing device is enabled to determine a concentration of the additional metal in the oil.

In another embodiment of the oil testing device, the electromagnetic radiation source is an Ultraviolet (UV) radiation source. The UV radiation source provides an easy and readily available means of exciting the quantum dot and the additional quantum dot.

The present technique is further described hereinafter with reference to illustrated embodiments shown in the accompanying drawings, in which:
- FIG 1: is a schematic representation of an exemplary embodiment of an oil testing device;
- FIG 2: is a schematic representation of an exemplary embodiment of an oil sample depicting a metal and an additional metal to be determined;
- FIG 3A: is a schematic representation of an exemplary embodiment of a quantum dot;
- FIG 3B: is a schematic representation of an exemplary embodiment of an additional quantum dot;
- FIG 4: is a schematic representation depicting working of the present technique when the metal to be determined is not present in the oil sample;
- FIG 5: is a schematic representation depicting working of the present technique when the metal to be determined is present in the oil sample;
- FIG 6: is a schematic representation depicting a set of reference intensities; and
- FIG 7: is a flow chart depicting an exemplary embodiment of an oil testing method, in accordance with aspects of the present technique.

Hereinafter, above-mentioned and other features of the present technique are described in details. Various embodiments are described with reference to the drawings, wherein like reference numerals are used to refer to like elements throughout. In the following description, for purpose of explanation, numerous specific details are set forth in order to provide a thorough understanding of one or more embodiments. It may be noted that the illustrated embodiments are intended to explain, and not to limit the invention. It may be evident that such embodiments may be practiced without these specific details.

The underlying idea of the invention is to use quantum dots to determine metals in oil samples. Quantum dots are nanocrystals made of semiconductor materials and display optical properties showing different colors that depend on the size of a given quantum dot. When excited using suitable energy, the quantum dot emits fluorescence of a frequency corresponding to the quantum dot. However, when a metal is present in close proximity of the quantum dot, the metal acts as a quencher and quenches the fluorescence emitted by the quantum dot due to florescence resonance energy transfer (FRET) between the quantum dot and the metal. This phenomenon of quenching is detectable in the fluorescence spectrum of the quantum dot. Thus by an occurrence or non-occurrence of the quenching phenomenon detectable in the florescence spectrum, the presence or absence of the metal is determined.

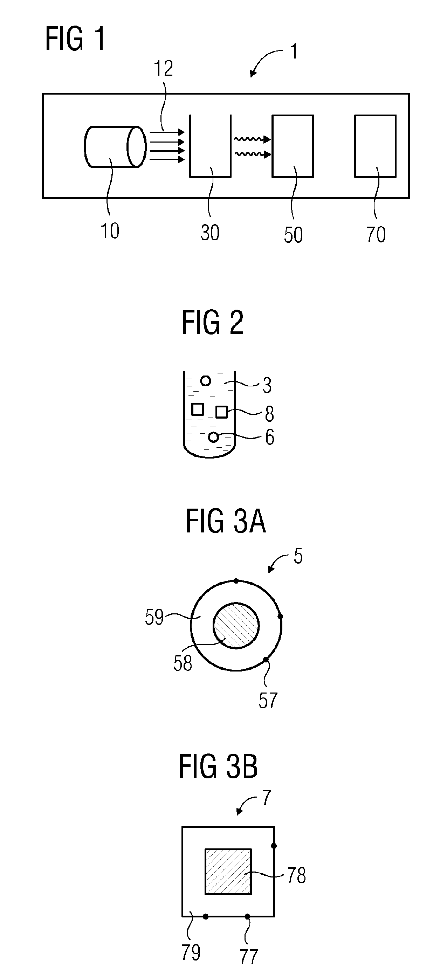

FIG 1 is a schematic representation of an exemplary embodiment of an oil testing device 1. FIG 2 is a schematic representation of an exemplary embodiment of an oil sample 3 depicting a metal 6 to be determined using the oil testing device 1. The oil sample 3 is obtained from lubricant oil used in a machine (not shown) such as a turbine, a gear, a compressor, a bearing, etc. The oil testing device 3 may optionally determine an additional metal 8 which may be present in the oil sample 3. Examples of the metal 6 and/or the additional metal 8 that are to be determined include, but not limited to, Iron (Fe), Copper (Cu), Lead (Pb), Tin (Sn), Nickel (Ni), Aluminum (Al), Cadmium (Cd), Silver (Ag), Titanium (Ti), Vanadium (V), and so forth.

FIG 3A and 3B schematically represent exemplary embodiments of a quantum dot 5 used to determine the metal 6 and an additional quantum dot 7 used to determine the additional metal 8, respectively.

The quantum dot 5 is adapted to specifically attach with the metal 6 that is to be detected in the oil sample 3. The quantum dot 5 and the metal 6 are capable of generating a fluorescence resonance energy transfer when the quantum dot 5 is attached with the metal 6. The quantum dot 5 and the additional quantum dot 7 are nanocrystals of semiconductor material. The quantum dot 5 includes a core 58 made up of a semiconductor material and may optionally include a shell 59 surrounding the core 58. Similarly, the additional quantum dot 7 includes a core 78 made up of a semiconductor material and may optionally include a shell 79 surrounding the core 78. Examples of semiconductor materials of the cores 58,78 may be, but not limited to, Cadmium selenide (CdSe), Cadmium sulfide (CdS), Indium arsenide (InAs), Indium phosphide (InP), Cadmium selenide sulfide, etc.

In an exemplary embodiment of the present technique, where the quantum dot 5 used does not include the shell 59, the core 58 is adapted to specifically bind to the metal 6. In an alternative embodiment, where the quantum dot 5 used includes the shell 59, the shell 59 is adapted to specifically bind to the metal 6. The shell 59 may comprise a binding material 57 having specific affinity towards the metal 6 and thus the binding material 57 of the quantum dot 5 specifically attaches to the metal 6 when the metal 6 is present in the surroundings of the quantum dot 5. The quantum dot 5 and the metal 6 are capable of generating a fluorescence resonance energy transfer (hereinafter referred to as FRET) when the quantum dot 5 is attached with the metal 6 and thus a quenching is observed in a fluorescence spectrum of the quantum dot 5. The attachment of the quantum dot 5 and the metal 6 may be through a chemical bond or by a physical proximity sufficient for occurrence of FRET wherein the quantum dot 5 is the donor and the metal 6 is the acceptor. The physical proximity may be a result of conformational or configuration characteristics of the quantum dot 5 and the metal 6.

Similarly, in another exemplary of the present technique, where the additional quantum dot 7 used does not include the shell 79, the core 78 is adapted to specifically bind to the metal 8. In an alternative embodiment, where the additional quantum dot 7 used includes the shell 79, the shell 79 is adapted to specifically bind to the metal 8. The shell 79 may comprise a binding material 77 having specific affinity towards the metal 8 and thus the binding material 77 of the additional quantum dot 7 specifically attaches to the additional metal 8 when the metal 8 is present in the surroundings of the additional quantum dot 7. The additional quantum dot 7 and the additional metal 8 are capable of generating a FRET when the additional quantum dot 7 is attached with the additional metal 8 and thus a quenching is observed in a fluorescence spectrum of the additional quantum dot 7. The attachment of the additional quantum dot 7 and the additional metal 8 may be through a chemical bond or by a physical proximity sufficient for occurrence of FRET wherein the additional quantum dot 7 is the donor and the additional metal 8 is the acceptor. The physical proximity may be a result of conformational or configuration characteristics of the additional quantum dot 7 and the additional metal 8.

The principle and techniques of fabrication of quantum dots, i.e. the quantum dot 5 and the additional quantum dot 7, is known in the art of nanocrystals and thus same has not been discussed herein for sake of brevity. An example of the quantum dot 5 is glutathione (GSH) shelled cadmium telluride (CdTe) nanocrystal (GSH/CdTe QD) which has a core 58 of CdTe material and a shell 59 of GSH. The GSH/CdTe QD specifically attaches to the metal 6, i.e. lead ion (Pb²⁺) for example. An example of the additional quantum dot 7 is thioglycerol shelled cadmium sulfide (CdS) nanocrystal (thiogycerol/CdS QD) which has a core 78 of CdS material and a shell 79 of thioglycerol. The thioglycerol/CdS QD specifically attaches to the additional metal 8, i.e. copper ion (Cu²⁺) for example.

Hereinafter, the present technique has been discussed for determination of the metal 6 using the quantum dot 5 (hereinafter referred to as QD 5), and thereafter the present technique has been shown briefly for the determination of the additional metal 8 by the additional quantum dot 7. Basically, the method for the determination of the additional metal 8 by the additional quantum dot 7 is equivalent to the method for the determination of the metal 6 using the quantum dot 5.

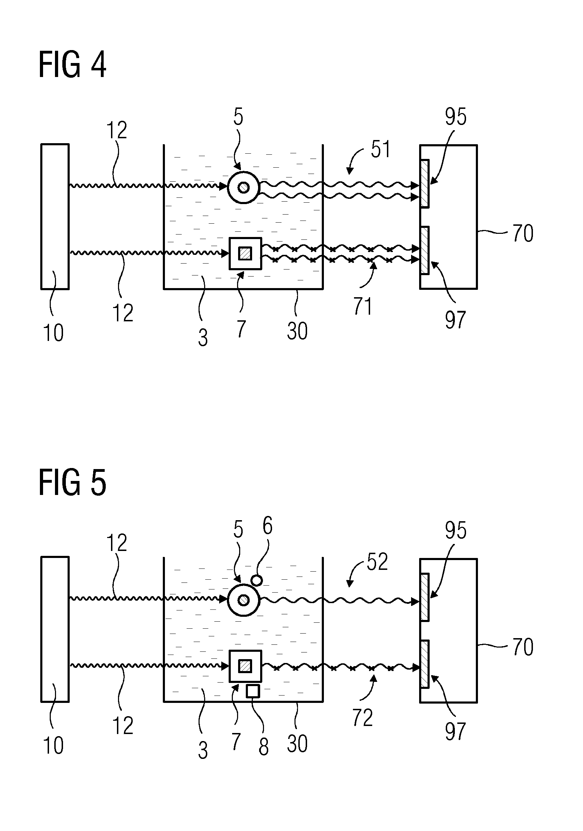

Referring to FIG 1 in combination with FIG 4 and FIG 5, the oil testing device 1 is used for determination of at least one metal 6 in the oil sample 3 by using the QD 5. The phrase 'determination of a/the metal' as used herein means determining or detecting a presence of the metal 6 in the oil sample 3 and may optionally include determining a concentration of the metal 6 in the oil sample 3.

The oil testing device 1 includes a sample port 30 for receiving the oil sample 3 and the QD 5, an electromagnetic radiation source 10, a detector 50 and a processor 70. The QD 5 may be present in a buffer solution. The oil sample 3 and the QD 5 buffer solution may be added to the sample port 30 simultaneously or successively.

The electromagnetic radiation source 10 generates an electromagnetic radiation 12 (hereinafter EM radiation 12). The electromagnetic radiation source 10 (hereinafter EM source 10) may be an Ultraviolet (UV) radiation source such as a UV laser source that generates the EM radiation 12, i.e. UV radiation. The EM radiation 12 is directed onto the oil sample 3 containing the QD 5. The EM radiation 12 may be directed onto the oil sample 3 in a variety of ways for example by directly transmitting the EM radiation 12 onto the oil sample 3 or by using a coupling lens or a beam deflector, in all of which cases the sample port 30 is such as to allow the transmission of the EM radiation 12 through the oil sample 3. Alternatively, the EM radiation 12 may be directed onto the oil sample 3 by using an optical fiber. The EM radiation 12 excites the QD 5 in the oil sample 3.

The detector 50 detects from the oil sample 3 a fluorescence spectrum 95 (shown in FIGs 4 and 5) corresponding to the QD 5 so excited. The detector 50 may be, but not limited to, a fluorescence spectrometer or a spectrofluorometer. For the present technique, FIG 4 schematically represents working of the technique when the metal 6 to be determined is not present in the oil sample 3 and FIG 5 schematically represents working of the technique when the metal 6 to be determined is present in the oil sample 3.

As shown in FIG 4, when the metal 6 is not present in the oil sample 3, the EM radiation 12 generated by the EM source 10 excites the QD 5 which in turn emits fluorescence in form of an electromagnetic signal 51 (hereinafter, signal 51) corresponding to the QD 5 that is detected by the detector 50 in form of the fluorescence spectrum 95 corresponding to the QD 5. The fluorescence spectrum 95 has a given signature such as an intensity of the signal 51 in absence of the metal 6. However, as shown in FIG 5, when the metal 6 is present in the oil sample 3, the EM radiation 12 generated by the EM source 10 excites the QD 5 which in turn emits fluorescence in form of an electromagnetic signal 52 (hereinafter, signal 52) corresponding to the QD 5 with attached metal 6. The signals 51, 52 are electromagnetic radiations having a particular wavelength. The fluorescence emitted by the QD 5 in presence of the metal 6 is quenched by occurrence of FRET. The EM signal 52 is detected by the detector 50 in form of or as a part of the fluorescence spectrum 95 corresponding to the QD 5 with attached metal 6. The fluorescence spectrum 95 in presence of the metal 6 has a different signature such as an intensity of the signal 52 as compared to the signature of the fluorescence spectrum 95 that includes the intensity of the signal 51 in absence of the metal 6. This difference is detected as a quenching in the fluorescence spectrum 95 corresponding to the QD 5. The quenching may be detected by comparing the signal 51 and the signal 52 and is defined to be present if the intensity of the signal 52 is lesser than the intensity of the signal 51. The metal 6 is determined by the quenching so determined in the fluorescence spectrum 95 corresponding to the QD 5.

Since the signature of the fluorescence spectrum 95 including the intensity of the signal 51 in absence of the metal 6 may be previously known under a known set of physical conditions, i.e. as a standard or reference or control signature of the fluorescence spectrum 95, an occurrence or absence of the quenching in the fluorescence spectrum 95 corresponding to the QD 5 may be determined only by a single detection of the fluorescence spectrum 95 by the detector 50. Thus if on testing an unknown oil sample, the fluorescence spectrum 95 detected contains a substantially same signature as a previously known standard or reference or control signature of the fluorescence spectrum 95, then the metal 6 is determined to be not present in the oil sample 3. Similarly, the signature of the fluorescence spectrum 95 including the intensity of the signal 52 in presence of the metal 6 may be previously known under a known set of physical conditions, i.e. as a positive standard signature of the fluorescence spectrum 95, and if on testing another unknown oil sample, the fluorescence spectrum 95 detected contains a substantially same signature as the previously known positive standard signature of the fluorescence spectrum 95, then the metal 6 is determined to be present in the oil sample 3.

The processor 50 determines the occurrence or the absence of the quenching in the fluorescence spectrum 95 corresponding to the QD 5 and thereby determines the presence or the absence of the metal 6 in the oil sample 3.

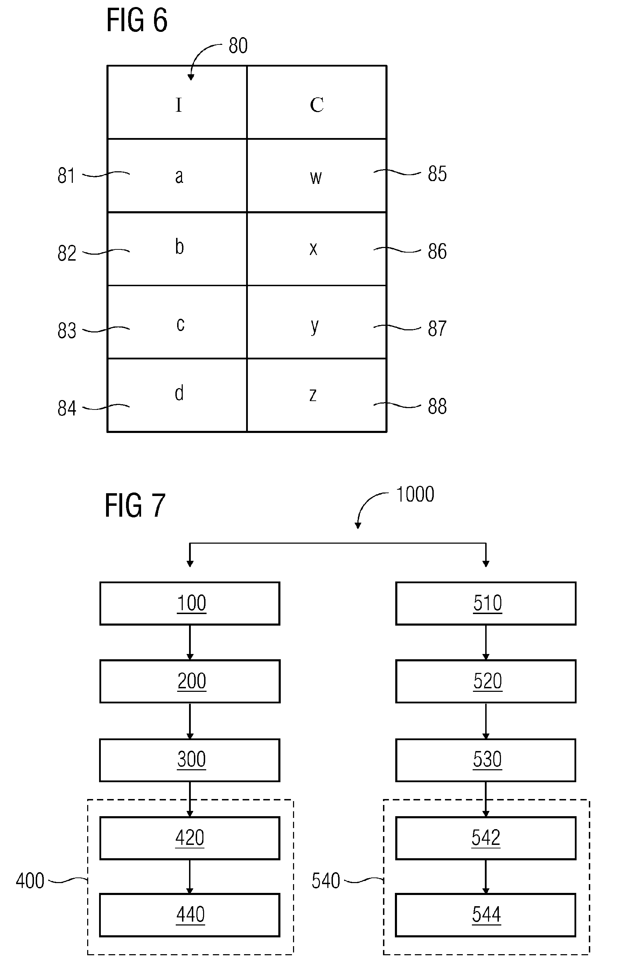

In another embodiment of the present technique where the phrase 'determination of a/the metal' includes determining a concentration of the metal 6 in the oil sample 3, the processor 70 in the oil testing device 1 identifies the intensity of the EM signal 52 in the fluorescence spectrum 95 corresponding to the QD 5. The EM signal 52 corresponds to the QD 5 attached to the metal 6. The processor 70 compares the intensity so identified with a set 80 of reference intensities 81,82,83,84 as depicted in FIG 6. The intensities 81,82,83,84 are tabulated under column 'I' in FIG 6. Each of the reference intensities 81,82,83,84 in the set 80 corresponds to a unique concentration value 85,86,87,88 of the metal 6 in the oil sample 3. The concentration values 85,86,87,88 are tabulated under column 'C' in FIG 6. The set 80 may be in form of a look-up table. The set 80 may be generated by testing different oil samples with varying known concentrations of the metal 6 using the QD 5 and storing the detected intensities against the different concentrations of the metal 6. These different detected intensities are used as the reference intensities 81,82,83,84.

Thus, if on testing an unknown oil sample the intensity of the signal 52 is detected as say 'n' units, and on comparing the 'n' units with the set 80, it is observed that 'n' units is same as 'c' units represented by reference numeral 83 in FIG 8, then a concentration 'y' unit represented by reference numeral 87 corresponding to the 'c' unit 83 is determined to be the concentration of the metal 6 in the tested unknown oil sample.

Hereinafter, the present technique has been shown briefly for the determination of the additional metal 8 by the additional quantum dot 7 (hereinafter referred to as AQD 7) with the help of FIGs 1 to 5. The determination of the additional metal 8 using the AQD 7 is similar to, and may be done simultaneously or successively with, the determination of the metal 6 using the QD 5 as explained above.

In order to determine the additional metal 8, the sample port 30 receives the AQD 7 which may be present in a suitable buffer solution same as or different from the buffer solution of the QD 5 and may be added to the sample port 30 simultaneously or successively with the QD 5 buffer solution and the oil sample 3. The AQD 7 in the oil sample 3 is excited by the EM radiation 12. A fluorescence spectrum 97 corresponding to the AQD 7 is detected at the detector 50. A quenching occurs when the additional metal 8 is present which is reflected in FIG 5 as compared to FIG 4, i.e. as a difference in intensities of an additional electromagnetic signal 72 (hereinafter, signal 72) of FIG 5 when the additional metal 8 is present as compared to that of an additional electromagnetic signal 71 (hereinafter, signal 71) of FIG 4 when the additional metal 8 is not present. The processor 70 then determines a quenching in the fluorescence spectrum 97 corresponding to the AQD 7 and in turn the additional metal 8 is determined by the quenching so determined in the fluorescence spectrum 97 corresponding to the AQD 7 in a similar way as done for the determination of the metal 6 using the QD 5. Thus the oil testing device 1 is enabled to determine a presence of the additional metal 8 in the oil sample 3.

Furthermore, a concentration of the additional metal 8 may be determined using the AQD 7 in a similar way as done for the determination of the concentration of the metal 6 using the QD 5. In this embodiment, the processor 70 in the oil testing device 1 identifies an additional intensity of the signal 72 in the fluorescence spectrum 97 corresponding to the AQD 7. The signal 72 corresponds to the AQD 7 attached to the additional metal 8. The processor 70 compares the additional intensity so identified with a set of additional reference intensities. Each of the additional reference intensities in the set corresponds to a unique concentration of the additional metal 8 in the oil sample 3.

The determination of the metal 6 and the additional metal 8 may be done simultaneously in furtherance of which the detector 70 detects the fluorescence spectrum 95 corresponding to the QD 5 and the fluorescence spectrum 97 corresponding to the AQD 7 simultaneously. Moreover, in another exemplary embodiment of the present technique in order to determine the metal 6 and the additional metal 8 simultaneously the QD 5 and the AQD 7 in the oil sample 3 are excited simultaneously using the EM radiation 12.

It may be noted that in accordance with aspects of the present invention, the oil testing device 1, may be made portable and miniaturized in form. The miniature oil testing device 1 includes a fluid micro-channel etched on a substrate layer such as glass to function as the sample port 30, an input optical fiber is adapted to receive the EM radiation 12 from an EM source 10 and to direct the EM radiation 12 to the oil sample 3 and the QD 5 and/or the QD 7 in the oil sample 3, and an output optical fiber is adapted to receive the emitted signals 51,52,71,72 and to direct the emitted signals 51,52,71,72 to the detector 50. The processor then detects the quenching in the fluorescence spectrums 95,97.

FIG 7, explained in combination with FIGs 1 to 6, represents a flow chart depicting an exemplary embodiment of an oil testing method 1000 (hereinafter the method 1000) for determination of at least one metal 6 in an oil sample 3, in accordance with aspects of the present technique. In the method 1000, the QD 5 is added to the oil sample 3 in a step 100. Subsequently, in a step 200, the QD 5 in the oil sample 3 is excited by an EM radiation 12 incident on the oil sample 3. Finally, from the oil sample 3 the fluorescence spectrum 95 corresponding to the QD 5 is detected in a step 300. As noted above, the metal 6 is determined by observing the quenching in the fluorescence spectrum 95 corresponding to the QD 5.

In an exemplary embodiment, the method 1000 further includes a step 400 of determining the concentration of the metal 6 in the oil sample 3. In the step 400, the intensity of the signal 52 in the fluorescence spectrum 95 corresponding to the QD 5 is identified in a step 420. Subsequently, in a step 440, the intensity so identified is compared with the set 80 of the reference intensities 81,82,83,84 and the concentration of the metal 6 in the oil sample 3 is determined as explained in reference to FIG 6.

In another embodiment, the method 1000 further includes a step 500 of determining the additional metal 8 in the oil sample 3. Although, FIG 7 depicts the step 500 as simultaneously performed with steps 100 to step 400, it may be noted that the step 500 may be successively, either before or after, performed with the steps 100 to 400. In the step 500, the AQD 7 is added to the oil sample 3 in a step 510. Subsequently, the AQD 7 in the oil sample 3 is excited by the EM radiation 12 incident on the oil sample 3 in a step 520. Finally, from the oil sample 3 the fluorescence spectrum 97 corresponding to the AQD 7 is detected in a step 530. As noted above, the additional metal 8 is determined by observing the quenching in the fluorescence spectrum 97 corresponding to the AQD 7.

In another embodiment, the method 1000 includes a step 540 of determining the concentration of the additional metal 8 in the oil sample 3. In performing the step 540, the additional intensity of the signal 72 in the fluorescence spectrum 97 corresponding to the AQD 7 is identified in a step 542. The signal 72 corresponds to the AQD 7 attached with the additional metal 8. Subsequently, in a step 544 the additional intensity so identified is compared with a set of additional reference intensities and thereby the concentration of the additional metal 8 in the oil sample 3 is determined in a way similar to the way of determining the concentration of the metal 6 as explained in reference to FIG 6.

While the present technique has been described in detail with reference to certain embodiments, it should be appreciated that the present technique is not limited to those precise embodiments. Rather, in view of the present disclosure which describes exemplary modes for practicing the invention, many modifications and variations would present themselves, to those skilled in the art without departing from the scope and spirit of this invention. The scope of the invention is, therefore, indicated by the following claims rather than by the foregoing description. All changes, modifications, and variations coming within the meaning and range of equivalency of the claims are to be considered within their scope.

## Claims

1. An oil testing method (1000) for determination of at least one metal (6) in an oil sample (3) obtained from a lubricant oil of a machine, the oil testing method (1000) comprising:
- adding (100) a quantum dot (5) to the oil sample (3), wherein the quantum dot (5) is adapted to specifically attach with the metal (6) and wherein the quantum dot (5) and the metal (6) are capable of generating a fluorescence resonance energy transfer when the quantum dot (5) is attached with the metal (6),
- exciting (200) the quantum dot (5) in the oil sample (3) by an electromagnetic radiation (12) incident on the oil sample (3), and
- detecting (300) from the oil sample (3) a fluorescence spectrum (95) corresponding to the quantum dot (5) so excited, wherein the metal (6) is determined by observing a quenching in the fluorescence spectrum (95) corresponding to the quantum dot (5).

2. The oil testing method (1000) according to claim 1, further comprising determining (400) a concentration of the metal (6) in the oil sample (3), wherein the determining (400) of the concentration of the metal (6) in the oil sample (3) comprises:
- identifying (420) an intensity of an electromagnetic signal (52) in the fluorescence spectrum (95) corresponding to the quantum dot (5), wherein the electromagnetic signal (52) corresponds to the quantum dot (5) attached with the metal (6), and
- comparing (440) the intensity so identified with a set (80) of reference intensities (81,82,83,84), wherein each of the reference intensities (81,82,83,84) in the set (84) corresponds to a unique concentration value (85,86,87,88) of the metal (6) in the oil sample (3).

3. The oil testing method (1000) according to claim 1 or 2, further comprising determining (500) an additional metal (8) in the oil sample (3), wherein the determining (500) of the additional metal (8) comprises:
- adding (510) an additional quantum dot (7) to the oil sample (3), wherein the additional quantum dot (7) is adapted to specifically attach with the additional metal (8) and wherein the additional quantum dot (7) and the additional metal (8) are capable of generating a fluorescence resonance energy transfer when the additional quantum dot (7) is attached with the additional metal (8),
- exciting (520) the additional quantum dot (7) in the oil sample (3) by the electromagnetic radiation (12) incident on the oil sample (3), and
- detecting (530) from the oil sample (3) a fluorescence spectrum (97) corresponding to the additional quantum dot (7) so excited, wherein the additional metal (8) is determined by observing a quenching in the fluorescence spectrum (97) corresponding to the additional quantum dot (7).

4. The oil testing method (1000) according to claim 3, wherein the quantum dot (5) and the additional quantum dot (7) are excited simultaneously.

5. The oil testing method (1000) according to claim 3 or 4, wherein the fluorescence spectrum (95) corresponding to the quantum dot (5) and the fluorescence spectrum (97) corresponding to the additional quantum dot (7) are detected simultaneously.

6. The oil testing method (1000) according to any of claims 3 to 5, further comprising determining (540) a concentration of the additional metal (8) in the oil sample (3), wherein the determining (540) of the concentration of the additional metal (8) in the oil sample (3) comprises:
- identifying (542) an additional intensity of an additional electromagnetic signal (72) in the fluorescence spectrum (97) corresponding to the additional quantum dot (7), wherein the additional electromagnetic signal (72) corresponds to the additional quantum dot (7) attached with the additional metal (8), and
- comparing (544) the additional intensity so identified with a set of additional reference intensities, wherein each of the additional reference intensities in the set corresponds to a unique concentration of the additional metal (8) in the oil sample (3).

7. The oil testing method (1000) according to any of claims 3 to 6, wherein the additional quantum dot (7) comprises a core (78) of semiconductor material and a shell (79) surrounding the core (78), wherein the shell (79) comprises a binding material (77) for specifically attaching with the additional metal (8).

8. The oil testing method (1000) according to any of claims 1 to 7, wherein the quantum dot (5) comprises a core (58) of semiconductor material and a shell (59) surrounding the core (58), wherein the shell (59) comprises a binding material (57) for specifically attaching with the metal (6).

9. The oil testing method (1000) according to any of claims 1 to 8, wherein the electromagnetic radiation (12) incident on the oil sample (3) is an Ultraviolet radiation.

10. An oil testing device (1) for determination of at least one metal (6) in an oil sample (3) by using a quantum dot (5) adapted to specifically attach with the metal (6), wherein the quantum dot (5) and the metal (6) are capable of generating a fluorescence resonance energy transfer when the quantum dot (5) is attached with the metal (6), the oil testing device (1) comprising:
- a sample port (30) for receiving the oil sample (3) and the quantum dot (5),
- an electromagnetic radiation source (10) adapted to generate an electromagnetic radiation (12) and to excite the quantum dot (5) in the oil sample (3) by directing the electromagnetic radiation (12) onto the oil sample (3), and
- a detector (50) for detecting from the oil sample (3) a fluorescence spectrum (95) corresponding to the quantum dot (5) so excited, and
- a processor (70) for determining a quenching in the fluorescence spectrum (95) corresponding to the quantum dot (5), wherein the metal (6) is determined by the quenching so determined in the fluorescence spectrum (95) corresponding to the quantum dot (5).

11. The oil testing device (1) according to claim 10, wherein the processor (70) is adapted:
- to identify an intensity of an electromagnetic signal (52) in the fluorescence spectrum (95) corresponding to the quantum dot (5) so detected, wherein the electromagnetic signal (52) corresponds to the quantum dot (5) attached to the metal (6), and
- to compare the intensity so identified with a set (80) of reference intensities (81,82,83,84), wherein each of the reference intensities (81,82,83,84) in the set corresponds to a unique concentration value (85,86,87,88) of the metal (5) in the oil sample (3).

12. The oil testing device (1) according to claim 10 or 11, the oil testing device (1) adapted to determine an additional metal (8) in the oil sample (3) by using an additional quantum dot (7) adapted to specifically attach with the additional metal (8), wherein the additional quantum dot (7) and the additional metal (8) are capable of generating a fluorescence resonance energy transfer when the additional quantum dot (7) is attached with the additional metal (8),
- wherein the sample port (30) is adapted to receive the additional quantum dot (7),
- wherein the electromagnetic radiation source (10) is adapted to excite the additional quantum dot (7) in the oil sample (3) by directing the electromagnetic radiation (12) onto the oil sample (3), and
- wherein the detector (50) is adapted to detect from the oil sample (3) a fluorescence spectrum (97) corresponding to the additional quantum dot (7) so excited, and
- wherein the processor (70) is adapted to determine a quenching in the fluorescence spectrum (97) corresponding to the additional quantum dot (7) and wherein the additional metal (8) is determined by the quenching so determined in the fluorescence spectrum (97) corresponding to the additional quantum dot (7).

13. The oil testing device (1) according to claim 12, wherein the detector (50) is adapted to detect the fluorescence spectrum (95) corresponding to the quantum dot (5) and to detect the fluorescence spectrum (97) corresponding to the additional quantum dot (7) simultaneously.

14. The oil testing device (1) according to claim 12 or 13, wherein the processor (70) is adapted:
- to identify an additional intensity of an additional electromagnetic signal (72) in the fluorescence spectrum (97) corresponding to the additional quantum dot (7), wherein the additional electromagnetic signal (72) corresponds to the additional quantum dot (7) attached to the additional metal (8), and
- to compare the additional intensity so identified with a set of additional reference intensities, wherein each of the additional reference intensities in the set corresponds to a unique concentration of the additional metal (8) in the oil sample (3).

15. The oil testing device (1) according to any of claims 10 to 14, wherein the electromagnetic radiation source (10) is an Ultraviolet radiation source.
